# EUROPÄISCHE PATENTSCHRIFT

(11) **EP 3 496 613 B1**
(45) Veröffentlichungstag und Bekanntmachung des Hinweises auf die Patenterteilung: **08.07.2020**
(21) Anmeldenummer: 17757459.7
(22) Anmeldetag: 01.08.2017
(51) Int. Cl.: A61B 5/15, A61B 5/151, A61B 5/157

(54) **ANORDNUNG ZUR ENTNAHME UND ZUR ANALYSE VON KÖRPERFLÜSSIGKEIT BEI PATIENTEN**
ASSEMBLY FOR COLLECTING AND ANALYZING BODY FLUID OF PATIENTS
SYSTÈME DE PRÉLÈVEMENT ET D'ANALYSE DES LIQUIDES CORPORELS DE PATIENTS

(30) Priorität: 09.08.2016 DE 102016114695
(43) Veröffentlichungstag der Anmeldung: 19.06.2019
(73) Patentinhaber: Pastötter, Albert, 83416 Saaldorf-Surheim (DE)
(72) Erfinder: Pastötter, Albert, 83416 Saaldorf-Surheim (DE)
(74) Vertreter: Naessens, Stephan
(86) Internationale Anmeldenummer: PCT/DE2017/100652
(87) Internationale Veröffentlichungsnummer: WO 2018/028744

(56) Entgegenhaltungen:
- EP-A1- 2 184 012
- DE-A1- 10 315 396
- US-A1- 2005 159 678
- US-A1- 2014 257 066

## Beschreibung

Die vorliegende Erfindung betrifft eine Anordnung zur Entnahme und zur Analyse von Körperflüssigkeiten eines Patienten gemäß der im Oberbegriff des Patentanspruches 1 näher definierten Art.

Es ist bekannt, dass zum Analysieren von Körperflüssigkeiten eines Patienten zunächst die entsprechende Körperflüssigkeit aus dem Körper des Patienten entnommen werden muss. Anschließend wird eine Analyse der entnommenen Körperflüssigkeit durchgeführt. Ein großes Anwendungsgebiet liegt bei der Blutzuckerwertbestimmung, insbesondere bei an Diabetes erkrankten Patienten. Da der Blutzuckerwert mehrmals täglich bestimmt werden muss, sind die Entnahme und die Analyse des selbst entnommenen Blutes in eigener Verantwortung durchzuführen. Hierzu gibt es entsprechende Lanzetten und Blutzuckermessgeräte. Der Patient kann sich mit der Lanzette selbst Blut entnehmen und diesen Bluttropfen einem Messbereich des Blutzuckermessgerätes zu führen, um dann den aktuellen Blutzuckerwert zu bestimmen.

Es hat sich gezeigt, dass es für sehbehinderte oder blinde Patienten aber auch für motorisch eingeschränkte Patienten schwierig bzw. unmöglich ist, dieses Verfahren bei sich selbst ohne Hilfe durchzuführen. Zum einen ist das Einstechen mit der Lanzette schwierig und zum anderen ergibt sich das Problem, dass nach dem Einstechen der entstandene Bluttropfen mit dem Messstreifen in Kontakt gebracht werden muss.

Beispielsweise aus der Druckschrift DE 103 15 396 A1 ist ein Blutzucker-Überwachungs-/Warnsystem bekannt. Das System umfasst einen Fingerling, ein Kontrollsteuergerät und Zuleitungen, wobei der Fingerling Messstreifen mit entsprechendem Glukoseenzym beinhaltet. Mit dem bekannten Blutzuckermesssystem wird eine Blutzuckermessung durchgeführt, indem das Messgerät in Handschuhformat ausgeführt ist, wobei ein Fingerling einen Finger einkleidet. Die Zuleitungen verbinden die Fingerkuppenstelle über Schlauchverbindungen mit der digitalen Messstation am Handgelenk und mit dem erforderlichen Pumpsystem zum Aufstauen des Blutes. Zudem ist eine Lanzette zum Anstechen der Fingerkuppe vorgesehen, die über das Kontrollgerät nach einem gewählten Intervall der Blutzuckermessung die Fingerkuppe ansticht, um so einen Bluttropfen zu gewinnen, um die Blutzuckerwertmessung vorzunehmen. Ferner muss eine korrekte Ausrichtung des Fingers an dem bekannten System vorgenommen werden, welches für Patienten mit Handicap nicht möglich ist.

Der vorliegenden Erfindung liegt die Aufgabe zu Grunde, eine Anordnung zur Entnahme und zum Analysieren von Körperflüssigkeit eines Patienten der eingangs beschriebenen Gattung vorzuschlagen, mit der auch für Patienten mit Handikap ein erfolgreicher Einsatz sichergestellt ist.

Diese Aufgabe wird erfindungsgemäß durch die Merkmale des Patentanspruches 1 gelöst, wobei sich vorteilhafte Ausgestaltungen aus den Unteransprüchen und der Beschreibung sowie den Zeichnungen ergeben.

Somit wird eine Anordnung zum Entnehmen und zum Analysieren von Körperflüssigkeit, vorzugsweise Blut bei einem Patienten, vorgeschlagen, wobei die Anordnung zumindest ein Analysegerät, zum Beispiel ein Blutzuckermessgerät oder dergleichen, und zumindest eine Lanzette umfasst. Um eine besonders einfache quasi automatische Entnahme und Analyse der Körperflüssigkeit mit der erfindungsgemäßen Anordnung nicht nur bei Patienten mit visuell eingeschränkter Wahrnehmung oder motorisch eingeschränkten Patienten zu realisieren, ist vorgesehen, dass die Lanzette und das Analysegerät einer Halteeinrichtung zum definierten Halten eines Körperabschnittes bezogen auf eine definierte Einstech- und Messstelle zugeordnet ist.

Dadurch, dass die Lanzette und das Analysegerät der Halteeinrichtung oder umgekehrt zugeordnet sind, wird einerseits die Lanzette zur Entnahme der Körperflüssigkeit und zum anderen auch das Analysegerät zum Erreichen und Analysieren der Körperflüssigkeit bzw. des Bluttropfens bezogen auf die Einstich- und Messstelle quasi automatisch justiert.

Demzufolge ist die vorgeschlagene Anordnung eine Stich-Mess-Hilfe, die es dem Anwender ermöglicht automatisch einzustechen und gezielt die Körperflüssigkeit aufzunehmen. Somit ist die Anordnung praktisch eine Zieleinrichtung bzw. ein Zielgerät, um den Messstreifen des Messgerätes exakt an die Einstichstelle zu bringen, wo sich die entnommene Körperflüssigkeit z. B. als Bluttropfen sammelt. Hierzu muss der Körper bzw. der Finger nicht bewegt werden, vielmehr wird der Messbereich des Analysegerätes durch die erfindungsgemäße Anordnung zielgenau zu der Einstichstelle und damit der Messstelle bewegt. Somit ermöglicht die erfindungsgemäße Anordnung eine Unterstützung für sehbehinderte Patienten und für Patienten mit eingeschränkter Motorik, sodass auch diese Patienten die Entnahme und Analyse der Körperflüssigkeit bzw. des Blutes selbst durchführen können. Die Anordnung kann mobil oder stationär eingesetzt werden. Es ist ohne weiteres möglich, dass die vorgeschlagene Anordnung auf Gegenständen befestigt wird, um weiter die Zielgenauigkeit zu verbessern.

Eine mögliche Ausführung der Erfindung kann vorgesehen, dass als Halteeinrichtung eine beispielsweise gehäuseförmige Aufnahme zum Einführen eines Fingers als Körperabschnitt vorgesehen ist, wobei eine Ausnehmung oder dergleichen als definierte Einstich- und Messstelle an einem Abschnitt der Aufnahme, z. B. bevorzugt der Oberseite oder Unterseite der Aufnahme, zugeordnet ist. Der in die Aufnahme eingeführte Finger wird quasi in die richtige Position zum Entnehmen des Blutes und zum Analysieren zwangsgeführt. Die Zwangsführung wird durch eine geführte Bewegung des Fingers realisiert. Dies kann beispielsweise durch eine geeignete Auflage bzw. Polsterung oder durch andere ähnliche Elemente realisiert werden, auf der der Finger beispielsweise angeordnet ist. Zur Zwangsführung wird eine Relativbewegung zwischen Auflage und Aufnahme bzw. Halteinrichtung ermöglicht, sodass der Finger im Inneren der Aufnahme zur Blutentnahme und zur Analyse korrekt positioniert wird.

Im Rahmen einer vorteilhaften Ausführung kann die Auflage bzw. Polsterung horizontal bewegbar im Innenraum der Aufnahme angeordnet sein. Die verschiedenen Positionen können zum Einstechen und Messen arretiert werden. Durch die Verstellbarkeit kann die Einstechstelle z. B. an dem Finger des Patienten variiert werden, sodass bei dem Patienten nicht bei jeder Messung dieselbe Hautstelle durch die Nadelspitze verletzt wird.

Beispielsweise ist es denkbar, dass die Lanzette als Einstichhilfe oder dergleichen über ein Halteelement an der Oberseite der Aufnahme schwenkbar und höhenverstellbar gehalten ist, sodass die Nadelspitze der Lanzette zum Einstechen an die Einstech- und Messstelle an der Oberseite der Aufnahme bewegbar ist. Das Halteelement kann als stabartiges Teil oder dergleichen ausgeführt werden, welches als Drehachse die Drehbewegung und auch die Höhenbewegung ermöglicht, sodass die Lanzette mit ihrer Nadelspitze durch die Drehbewegung einen Radius beschreibt, auf dem die Ausnehmung als definierte Einstichstelle auf der Oberseite der Aufnahme liegt. Wenn die Einstichstelle erreicht ist, wird beispielsweise durch einfaches Drücken die Nadelspitze zu der Einstichstelle bewegt, sodass durch das Einstechen in die Hautoberfläche des in der Halteeinrichtung vorgesehenen Fingers ein Austreten von Blut erzeugt wird. Das Analysegerät kann über das gleiche oder ein separates Halteelement an der Oberseite der Aufnahme schwenkbar und höhenverstellbar gehalten werden, sodass der Messbereich des Analysegerätes zum Messen an die Einstich- und Messstelle bewegbar ist. Wenn die Messstelle erreicht ist, kann durch eine Höhenbewegung beispielsweise durch Drücken des Analysegerätes der Messbereich des Analysegerätes zielgenau zu der Einstich- und Messstelle bewegt werden, sodass sichergestellt ist, dass der Messbereich in Kontakt mit der entnommenen Körperflüssigkeit, zum Beispiel einem Bluttropfen, kommt. Auf diese Weise kann die Analyse des Blutes quasi automatisch durchgeführt werden. Bei dieser Art der Anordnung wird der Finger mit der Einstich-und Messstelle definiert ortsfest gehalten, wobei die Lanzette und das Analysegerät entsprechend zwangsbewegt wird, um zielgenau zur Einstech- und Messstelle bewegt zu werden.

Eine konstruktiv einfachere Ausführung kann dadurch realisiert werden, dass lediglich das Analysegerät an dem Halteelement drehbar bzw. schwenkbar und höhenverstellbar gehalten ist und die Einstichhilfe bzw. Lanzette direkt an der Ausnehmung der Halteeinrichtung an der Einstichstelle justiert wird. Hierzu kann beispielsweise vorgesehen sein, dass der Ausnehmung bzw. der Einstich- und Messstelle ein Einführadapter oder dergleichen zum definierten Halten der Lanzette vorgesehen ist. Auf diese Weise ist jedenfalls ein automatisches zielgenaues Entnehmen bzw. Einstechen und ein automatisches Analysieren des entnommenen Blutes ohne weiteres auch für Patienten möglich.

Eine weitere Ausführung der Erfindung kann vorsehen, dass das Analysegerät und die Lanzette über das Halteelement schwenkbar in der Halteeinrichtung unterhalb der Aufnahme gehalten ist, sodass die Nadelspitze der Lanzette und der Messbereich des Analysegerätes der definierten Einstich- und Messstelle zuordenbar ist, wobei als definierte Einstich- und Messstelle an der Unterseite der Aufnahme die Ausnehmung zugeordnet ist. Ferner kann vorgesehen sein, dass die Aufnahme über entsprechende Lagerelemente höhenverstellbar in bzw. an der Halteeinrichtung gelagert ist, sodass die Einstich- und Messstelle an die Nadelspitze der Lanzette zum Einstechen und an den Messbereich des Analysegerätes zum Messen bewegbar ist. Somit wird bei dieser Ausführung die Aufnahme z. B. mit dem enthaltenen Finger bevorzugt nach unten bewegt und durch entsprechendes Ausrichten bzw. Schwenken der Lanzette der Bluttropfen durch das Einstechen erzeugt. Anschließend wird durch entsprechendes Drehen bzw. Schwenken des Haltelements das Analysegerät mit dem Messbereich ausgerichtet, wobei der Messbereich durch Herunterdrücken der Aufnahme mit dem Bluttropfen in Kontakt gebracht. Bei dieser Art der Anordnung werden die Lanzette und das Analysegerät lediglich schwenkbar bzw. drehbar angeordnet, wobei die Aufnahme mit dem Finger höhenverstellbar gelagert ist. Somit sind die Aufnahme bezogen auf die Höhenbewegung zwangsgeführt und das Analysegerät und die Lanzette bezogen auf die Drehbewegung zwangsgeführt.

Als Lanzette kann beispielsweise eine Einmal- oder auch eine wiederverwendbare Einstichhilfe verwendet werden. Hierbei ist die Verwendung von herkömmlichen auf dem Markt verfügbaren Lanzetten ohne weiteres möglich, da keine speziellen Anforderungen notwendig sind. Es sind auch andere Stechhilfen einsetzbar Ebenso kann jedes herkömmliche Analysegerät, beispielsweise Blutzuckermessgerätes mit einem Messbereich zum Beispiel als Messstreifen oder dergleichen eingesetzt werden.

Die vorliegende Erfindung wird anhand der Zeichnungen weiter erläutert. Es zeigen:
Figur 1 eine schematische dreidimensionale Ansicht einer ersten möglichen Ausführungsvariante einer Anordnung zum Entnehmen und Analysieren einer Körperflüssigkeit eines Patienten;
Figur 2 eine Draufsicht auf die Anordnung gemäß Figur 1;
Figur 3 eine schematische Seitenansicht einer zweiten möglichen Ausführungsvariante der Anordnung; und
Figur 4 eine schematische dreidimensionale Ansicht einer dritten möglichen Ausführungsvariante der Anordnung.

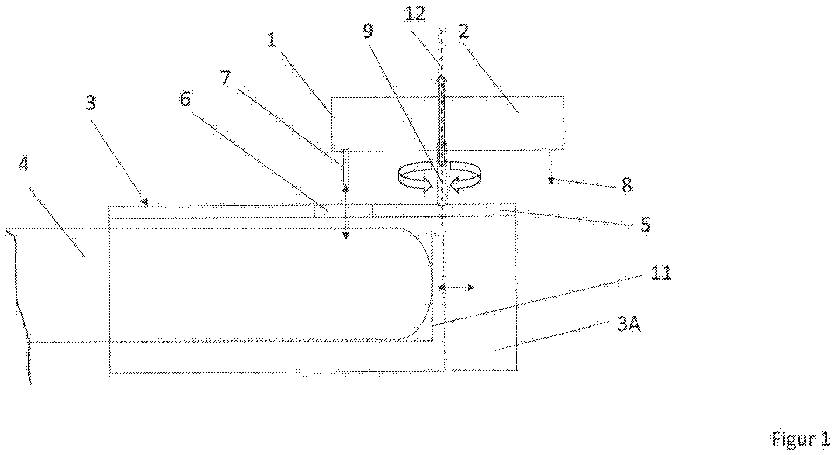

In den Figuren 1 bis 4 sind beispielhaft zwei mögliche Ausführungsvarianten einer erfindungsgemäßen Anordnung zum Entnehmen und Analysieren einer Körperflüssigkeit eines Patienten exemplarisch gezeigt.

Unabhängig von den jeweiligen Ausführungsvarianten umfasst die vorgeschlagene Anordnung zur Entnahme und zum Analysieren von Körperflüssigkeiten, insbesondere Blut, eines Patienten zumindest ein Analysegerät 1 und zumindest eine Lanzette 2. Erfindungsgemäß ist vorgesehen, dass der Lanzette 2 und dem Analysegerät 1 eine Halteeinrichtung 3 zum definierten Halten eines Körperabschnittes bzw. eines Fingers 4 bezogen auf eine definierte Einstich-und Messstelle zugeordnet ist.

Wie aus den Figuren 1, 3 und 4 ersichtlich ist, umfasst die Halteeinrichtung 3 eine gehäuseförmige Aufnahme 3A, 3B zum Einführen eines Fingers 4 des Patienten. Ein bevorzugtes Ziel der Erfindung ist es somit durch die Anordnung sehbehinderte Menschen und Menschen mit eingeschränkter Motorik bei der Entnahme und der Analyse des Blutes derart zu unterstützen, dass diese Patienten selbstständig zum Beispiel Blutzuckermessungen durchführen können. Die Anordnung kann an Gegenständen, wie zum Beispiel an einem Tisch befestigt werden. Auf diese Weise ist ein stationärer aber auch mobiler Einsatz möglich.

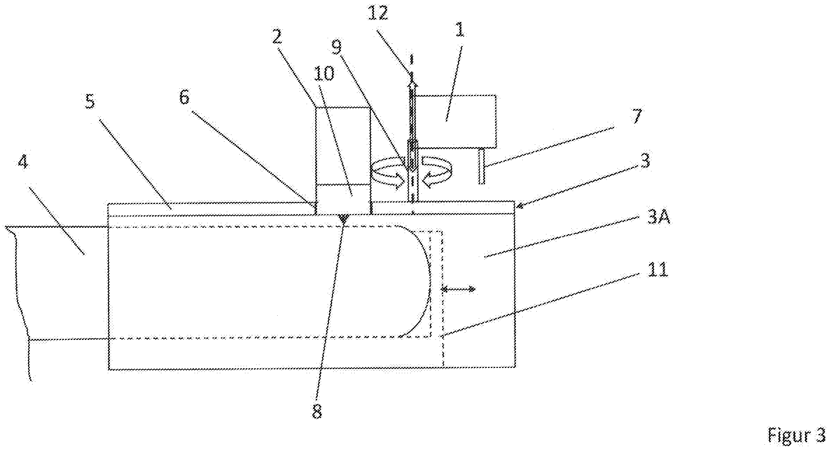

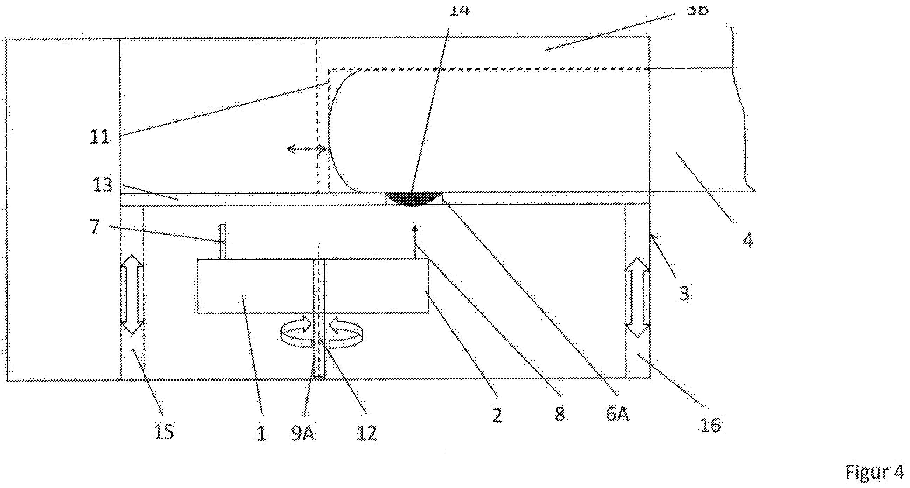

Die gehäuseförmige Aufnahme 3A, 3B weist im Innenraum eine Polsterung 11 zum definierten Halten des Fingers 4 bezogen auf die Einstich- und Messstelle. In Figur 1 und 3 ist als definierte Einstich- und Messstelle eine Ausnehmung 6 an der Oberseite 5 der Aufnahme 3A zugeordnet ist. Bei der Ausführung gemäß Figur 4 ist als definierter Einstich- und Messstelle die Ausnehmung 6A an der Unterseite 13 der Aufnahme 3B vorgesehen. Unabhängig von der jeweiligen Ausführung kann der jeweilige Patient seinen Finger 4 in den Innenraum der Aufnahme 3A, 3B einführen und in vordefinierter Lage auf der Polsterung 11 platzieren. Auf diese Weise ist sichergestellt, dass der Finger 4 bei sehbehinderten Patienten korrekt zur Blutentnahme und zur Analyse ausgerichtet ist. Die Polsterung 11 ist in der Aufnahme 3A, 3B horizontal bewegbar im Innenraum angeordnet, welches durch einen Doppelpfeil in den Figuren 1,3 und 4 angedeutet ist. Hierdurch ist es für den Patienten möglich, die definierte Anordnungsposition zu verändern, sodass nicht immer die gleiche Hautoberfläche des Fingers als Einstich- und Messstelle vorgesehen ist.

Gemäß Figur 1 ist bei der ersten Ausführungsvariante vorgesehen, dass das Analysegerät 1 und die Lanzette 2 derart bewegbar an der Aufnahme 3A, 3B bzw. in der Halteeinrichtung 3 befestigt sind, dass ein Messbereich 7 des Analysegerätes 1 und eine Nadelspitze 8 der Lanzette 2 der definierten Einstech-und Messstelle bzw. der Ausnehmung 6 der Aufnahme 3A, 3B nacheinander zugeordnet werden kann. Durch diese quasi automatische Zuordnung sind eine Entnahme und auch eine Analyse ohne weiteres möglich.

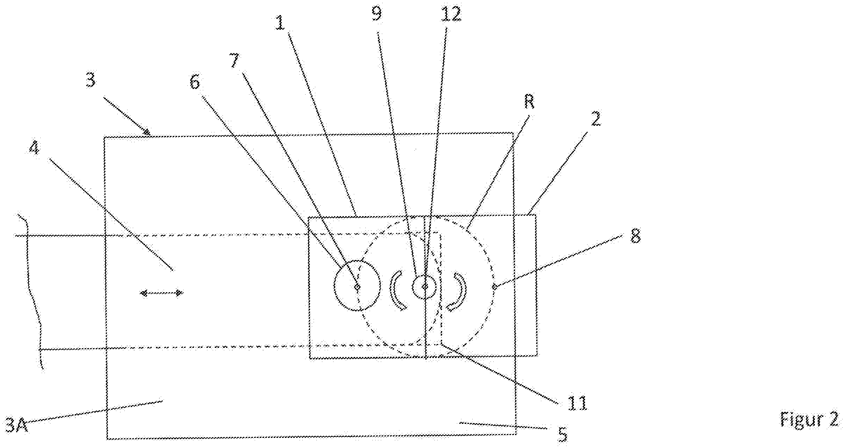

Wie insbesondere aus den Figuren 1 und 2 ersichtlich ist, wird die Lanzette 2 über ein Halteelement 9 an der Oberseite 5 der Aufnahme 3A, 3B schwenkbar und höhenverstellbar gehalten, welches durch entsprechende Pfeile angedeutet ist. Auf diese Weise ist die Nadelspitze 8 der Lanzette 2 zum Einstechen an die Einstich- und Messstelle und damit an die Ausnehmung 6 bewegbar. Um die Höhenverstellung bzw. die Höhenbewegung abzudämpfen, kann ein entsprechender Dämpfer an dem Halteelement 9 vorgesehen sein. Ferner ist das Analysegerät 1 ebenfalls über das Halteelement 9 an der Oberseite 5 der Aufnahme 3A, 3B schwenkbar und höhenverstellbar gehalten, sodass der Messbereich 7 des Analysegerätes 1 zum Messen bzw. Analysieren an die Einstich- und Messstelle bzw. an die Ausnehmung 6 bewegbar.

Eine konstruktiv einfache Ausführung ist somit in den Figuren 1 und 2 dargestellt, bei der das Halteelement 9 eine Doppelfunktion übernimmt, da an dem Halteelement 9 quasi gegenüberliegend das Analysegerät 1 und die Lanzette 2 angeordnet sind.

Wie insbesondere aus Figur 2 ersichtlich ist, ist der Drehpunkt des Halteelements 9 derart ausgerichtet, dass sowohl der Messbereich 7 als auch die Nadelspitze 8 und die Ausnehmung 6 mit dem Einstech-und Messbereich auf einem gemeinsamen Radius R liegen. Demnach ist die Drehachse 12 des Halteelements 9 derart angeordnet ist, dass auf dem Radius R um die Drehachse 12 die Einstech- und Messstelle bzw. Ausnehmung 6, der Messbereich 7 und die Nadelspitze 8 angeordnet sind. Dadurch ist sichergestellt, dass bei einer Drehung des Halteelements 9 sowohl der Messbereich 7 als auch die Nadelspitze 8 die Ausnehmung 6 und damit die definierte Einstich- und Messstelle zielgenau erreichen.

In Figur 3 ist eine zweite Ausführungsvariante der Anordnung dargestellt, bei der lediglich das Analysegerät 1 an dem Halteelement 9 schwenkbar und höhenverstellbar gehalten ist. Zum Entnehmen des Blutes aus dem Finger 4 ist der Ausnehmung 6 ein Einführadapter 10 zugeordnet, mit dem ein definiertes Halten der Lanzette 2 ermöglicht wird. Die Lanzette 2 wird durch den Einführadapter 10 zielgenau an der Einstich- und Messstelle gehalten und es kann durch einfaches Niederdrücken der Lanzette 2 die Nadelspitze 8 in die Hautoberfläche des Fingers 4 eintreten und somit den Bluttropfen erzeugen. Anschließend wird die Lanzette 2 entnommen und das Analysegerät 1 mit dem Messbereich 7 zu der Einstech- und Messstelle geschwenkt und anschließend niedergedrückt, um den Messbereich 7 mit dem entnommenen Blut in Berührung zu bringen und dadurch die Analyse zu starten.

Mit der vorgeschlagenen Anordnung kann wie folgt die Entnahme und die Analyse der Körperflüssigkeit eines Patienten durchgeführt werden. Zunächst führt der Patient einen beliebigen Finger 4 in den Innenraum der gehäuseförmigen Aufnahme 3A, 3B und positioniert diesen Finger 4 aufgrund der Polsterung 11 zielgenau unterhalb der Einstech-und Messstelle bzw. der Ausnehmung 6. Zur Entnahme des Blutes wird entweder die an dem Halteelement 9 montierte Lanzette 2 geschwenkt und abgesenkt, um zielgenau durch die Ausnehmung 6 in die Hautoberfläche des Fingers 4 mit der Nadelspitze 8 einzustechen. Alternativ kann eine separate Lanzette 2 auch durch den Einführadapter zielgenau positioniert werden und anschließend die Nadelspitze 8 abgesenkt werden, um in die Hautoberfläche einstechen. Zur Blutzuckermessung wird anschließend das Analysegerät 1, welches an dem Halteelement 9 montiert ist und mit einem Messstreifen als Messbereich 7 bestückt ist, durch Schwenken und Absenken des Analysegerätes 1 zielgenau zu der Ausnehmung 6 geführt, um den entnommenen Bluttropfen zu berühren.

In Figur 4 ist eine dritte Ausführungsvariante der Erfindung dargestellt. Bei dieser Ausführungsvariante ist das Analysegerät 1 und die Lanzette 2 über ein Halteelement 9A schwenkbar unterhalb der Aufnahme 3B in der Halteeinrichtung 3 gehalten, die quasi als Haltegestell dient, sodass die Nadelspitze 8 der Lanzette 2 und ein Messbereich 7 des Analysegerätes 1 der definierten Einstich- und Messstelle zuordenbar ist. Als definierte Einstich- und Messstelle ist an der Unterseite 13 der Aufnahme 3B eine Ausnehmung 6A angeordnet. Die Aufnahme 3B ist über längenverstellbare Lagerstangen 15,16 höhenverstellbar an der Halteeinrichtung 3 gelagert, sodass die Einstich- und Messstelle an die Nadelspitze 8 der Lanzette 2 zum Einstechen und anschließend an den Messbereich 7 des Analysegerätes 1 zum Messen bewegbar ist. Auch bei dieser Ausführung ist die Drehachse 12 des Halteelements 9A derart ausgerichtet, dass auf einem Radius R um die vertikale Drehachse 12 die Einstich- und Messstelle bzw. Ausnehmung 6A, der Messbereich 7 und die Nadelspitze 8 angeordnet sind. Auf diese Weise ist bei dieser Ausführung die Aufnahme 3B höhenverstellbar über die Lagerstangen 15,16 angeordnet, während die Lanzette 2 und die das Analysegerät 1 lediglich schwenkbar bzw. drehbar um die vertikale Drehachse 12 des Halteelements 9A angeordnet sind.

Mit der vorgeschlagenen Ausführungsvariante kann wie folgt die Blutentnahme und die Analyse der Körperflüssigkeit bei einem Patienten durchgeführt werden. Zunächst führt der Patient seinen Finger 4 in den Innenraum der gehäuseförmige Aufnahme 3B und positioniert diesen Finger 4 aufgrund der Polsterung 11 zielgenau oberhalb der Ausnehmung 6A. Zur Entnahme des Blutes wird die Aufnahme 3B mit dem Finger nach unten gedrückt, wobei vorher die Lanzette 2 durch entsprechendes Schwenken der Ausnehmung 6A zugeordnet ist. Zielgenau wird die Nadelspitze 8 in die Hautoberfläche des Fingers 4 eingestochen. Anschließend wird die Aufnahme 3B beispielsweise federbelastet wieder nach oben geführt und anschließend das Analysegerät 1 mit dem Messbereich 7 durch entsprechendes Schwenken der Ausnehmung 6A zugeordnet. Durch entsprechendes Runterdrücken der Aufnahme 3B wird der an der Einstich- und Messstelle erzeugte Bluttropfen 14 mit dem Messbereich 7 des Analysegerätes 1 in Kontakt gebracht, um die Messung bzw. die Analyse durchführen zu können. Bei dieser Ausführungsvariante ergibt sich unter anderem der Vorteil, dass aufgrund der Schwerkraft die Bildung des Bluttropfens 14 erleichtert ist. Der Bluttropfen 14 hängt quasi nach unten aus der Ausnehmung 6A heraus.

Wenn beispielsweise ein Farb- oder Flüssigkeitserkennungssensor oder dergleichen montiert ist, kann dieser zum Beispiel akustisch signalisieren, wenn ein Bluttropfen 14 an der Einstech- und Messstelle bzw. an der Ausnehmung 6,6 A vorhanden ist. Ferner wird der Patient zum Beispiel akustisch darüber informiert, ob die Messung erfolgreich durchgeführt werden konnte.

### Bezuqszeichen

- 1: Analysegerät
- 2: Lanzette
- 3: Halteeinrichtung
- 3A,3B: gehäuseförmige Aufnahme
- 4: Finger des Patienten
- 5: Oberseite der Aufnahme
- 6,6A: Ausnehmung bzw. Einstich- und Messstelle
- 7: Messbereich
- 8: Nadelspitze
- 9, 9A: Halteelement
- 10: Einführadapter
- 11: Polsterung
- 12: Drehachse des Halteelements
- 13: Unterseite der Aufnahme
- 14: Bluttropfen
- 15: längenverstellbare Lagerstange
- 16: längenverstellbare Lagerstange
- R: Radius

## Patentansprüche

1. Anordnung zur Entnahme und zum Analysieren von Körperflüssigkeit eines Patienten, mit zumindest einem Analysegerät (1) und mit zumindest einer Lanzette (2), wobei die Lanzette (2) und das Analysegerät (1) einer Halteeinrichtung (3) zum definierten Halten eines Körperabschnittes bezogen auf eine definierte Einstich- und Messstelle zugeordnet sind, wobei die Halteeinrichtung (3) eine gehäuseförmige Aufnahme (3A, 3B) zum Einführen eines Fingers (4) als Körperabschnitt aufweist, wobei die gehäuseförmige Aufnahme (3A, 3B) im Innenraum eine Auflage zum definierten Halten des Fingers (4) bezogen auf die Einstich- und Messstelle aufweist, **dadurch gekennzeichnet, dass** eine Relativbewegung zwischen Auflage und Aufnahme (3A, 3B) vorgesehen ist, sodass der Finger im Inneren der Aufnahme (3A, 3B) der definierten Einstich- und Messstelle zugeordnet ist.

2. Anordnung nach Anspruch 1, **dadurch gekennzeichnet, dass** eine Polsterung (11) als Auflage horizontal bewegbar im Innenraum der Aufnahme (3A, 3B) angeordnet ist.

3. Anordnung nach einem der vorangehenden Ansprüche, **dadurch gekennzeichnet, dass** das Analysegerät (1) und die Lanzette (2) bewegbar an der Oberseite (5) der Aufnahme (3A) befestigt sind, sodass eine Nadelspitze (8) der Lanzette (2) und ein Messbereich (7) des Analysegerätes (1) der definierten Einstich- und Messstelle zuordenbar ist, wobei als definierte Einstich- und Messstelle an der Oberseite (5) der Aufnahme (3A) eine Ausnehmung (6) zugeordnet ist.

4. Anordnung nach Anspruch 3, **dadurch gekennzeichnet, dass** die Lanzette (2) und das Analysegerät (1) über ein Halteelement (9) an der Oberseite (5) der Aufnahme (3A) schwenkbar und höhenverstellbar gehalten ist, sodass die Nadelspitze (8) der Lanzette (2) zum Einstechen an die Einstich- und Messstelle und der Messbereich (7) des Analysegerätes (1) zum Messen an die Einstich- und Messstelle bewegbar ist.

5. Anordnung nach einem der Ansprüche 1 oder 2, **dadurch gekennzeichnet, dass** das Analysegerät (1) und die Lanzette (2) über ein Halteelement (9A) schwenkbar um eine vertikale Drehachse (12 in der Halteeinrichtung (3) unterhalb der Aufnahme (3B) gehalten ist, sodass eine Nadelspitze (8) der Lanzette (2) und ein Messbereich (7) des Analysegerätes (1) der definierten Einstich- und Messstelle zuordenbar ist, wobei als definierte Einstich- und Messstelle an der Unterseite (13) der Aufnahme (3B) eine Ausnehmung (6A) zugeordnet ist.

6. Anordnung nach Anspruch 3, **dadurch gekennzeichnet, dass** die Aufnahme (3B) über längenverstellbare Lagerstangen (15, 16) höhenverstellbar an der Halteeinrichtung (3) gelagert ist, sodass die Einstich- und Messstelle an die Nadelspitze (8) der Lanzette (2) zum Einstechen und an den Messbereich (7) des Analysegerätes (1) zum Messen bewegbar ist.

7. Anordnung nach einem der vorangehenden Ansprüche, **dadurch gekennzeichnet, dass** die Drehachse (12) des Halteelements (9, 9A) derart ausgerichtet ist, dass auf einem Radius (R) um die vertikale Drehachse (12) die Einstich- und Messstelle bzw. Ausnehmung (6, 6A), der Messbereich (7) und die Nadelspitze (8) angeordnet sind.

8. Anordnung nach einem der vorangehenden Ansprüche, **dadurch gekennzeichnet, dass** der Einstich- und Messstelle ein Einführadapter (10) zum definierten Halten der Lanzette (2) zugeordnet ist.

9. Anordnung nach einem der vorangehenden Ansprüche, **dadurch gekennzeichnet, dass** eine Farb- und/oder Flüssigkeitserkennungseinrichtung der Einstich- und Messstelle zugeordnet ist.

10. Anordnung nach einem der vorangehenden Ansprüche, **dadurch gekennzeichnet, dass** als Analysegerät (1) ein Blutzuckermessgerät zum Messen des Blutzuckerwertes vorgesehen ist.

## Claims

1. An arrangement for collecting and analyzing body fluid of a patient, with at least one analyzer (1) and with at least one lancet (2), the lancet (2) and the analyzer (1) being associated with a holding means (3) for the defined holding of a body portion relative to a defined puncture and measurement site, wherein said holding means (3) has a housing-shaped receptacle (3A, 3B has) for insertion of a finger (4) as a body portion, the housing-shaped receptacle (3A, 3B) having a support in the interior for a defined holding of the finger (4) relative to the puncture and measurement site, **characterized in that** a relative movement between the support and the receptacle (3A, 3B) is provided so that the finger inside the receptacle (3A , 3B) is associated with the defined puncture and measurement site.

2. The arrangement according to claim 1, **characterized in that** a padding (11) is arranged horizontally movable as a support in the interior of the receptacle (3A, 3B).

3. The arrangement according to any one of the preceding claims, **characterized in that** the analyzer (1) and the lancet (2) are movably attached to the top (5) of the receptacle (3A), so that a needle tip (8) of the lancet (2) and a measurement area (7) of the analyzer (1) can be associated with the defined puncture and measurement site, a recess (6) being associated as a defined puncture and measurement site on the top (5) of the receptacle (3A).

4. The arrangement according to claim 3, **characterized in that** the lancet (2) and the analyzer (1) are held pivotally and height-adjustably via a holding element (9) on the top (5) of the receptacle (3A), so that the needle tip (8) of the lancet (2) is movable to the puncture and measurement site for piercing and the measurement area (7) of the analyzer (1) is moveable to the puncture and measurement site for measuring.

5. The arrangement according to any one of claims 1 or 2, **characterized in that** the analyzer (1) and the lancet (2) are held pivotably about a vertical axis of rotation (12) via a holding element (9A) in the holding means (3) below the receptacle (3B) so that a needle tip (8) of the lancet (2) and a measurement area (7) of the analyzer (1) can be associated with the defined puncture and measurement site, a recess (6A) being associated as a defined puncture and measurement site on the bottom (13) of the receptacle (3B).

6. The arrangement according to claim 3, **characterized in that** the receptacle (3B) is supported height-adjustably on the holding means (3) via length-adjustable support bars (15, 16) so that the puncture and measurement site is moveable to the needle tip (8) of the lancet (2) for piercing and to the measurement area (7) of the analyzer (1) for measuring.

7. The arrangement according to any one of the preceding claims, **characterized in that** the axis of rotation (12) of the holding element (9, 9A) is oriented in such a way that the puncture and measurement site and the recess (6, 6A) respectively, the measurement area (7) and the needle tip (8) are arranged on a radius (R) about the vertical axis of rotation (12).

8. The arrangement according to any one of the preceding claims, **characterized in that** an insertion adapter (10) for the defined holding of the lancet (2) is associated with the puncture and measurement site.

9. The arrangement according to any one of the preceding claims, **characterized in that** a color and/or fluid recognition means is associated with the puncture and measurement site.

10. The arrangement according to any one of the preceding claims, **characterized in that** a blood glucose meter is provided as an analyzer (1) for measuring the blood glucose level.

## Revendications

1. Agencement pour le prélèvement et l'analyse de liquide corporel d'un patient, avec au moins un appareil d'analyse (1) et avec au moins une lancette (2), la lancette (2) et l'appareil d'analyse (1) étant associés à un dispositif de maintien (3) pour le maintien défini d'une partie du corps par rapport à un emplacement de piqûre et de mesure défini, le dispositif de maintien (3) présentant un logement (3A, 3B) en forme de boîtier pour insérer un doigt (4) comme partie du corps, le logement (3A, 3B) en forme de boîtier présentant dans l'espace intérieur un appui pour le maintien défini du doigt (4) par rapport à l'emplacement de piqûre et de mesure, **caractérisé en ce qu'**un mouvement relatif entre l'appui et le logement (3A, 3B) est prévu de sorte que le doigt à l'intérieur du logement (3A, 3B) soit associé à l'emplacement de piqûre et de mesure défini.

2. Agencement selon la revendication 1, **caractérisé en ce qu'**un capitonnage (11) mobile horizontalement est disposé comme appui dans l'espace intérieur du logement (3A, 3B).

3. Agencement selon l'une quelconque des revendications précédentes, **caractérisé en ce que** l'appareil d'analyse (1) et la lancette (2) sont fixés de manière mobile sur le côté supérieur (5) du logement (3A) de sorte qu'une pointe d'aiguille (8) de la lancette (2) et une zone de mesure (7) de l'appareil d'analyse (1) puissent être associés à l'emplacement de piqûre et de mesure défini, un évidement (6) étant associé en tant qu'emplacement de piqûre et de mesure défini sur le côté supérieur (5) du logement (3A).

4. Agencement selon la revendication 3, **caractérisé en ce que** la lancette (2) et l'appareil d'analyse (1) sont maintenus de manière pivotante et réglables en hauteur au moyen d'un élément de maintien (9) sur le côté supérieur (5) du logement (3A) de sorte que la pointe d'aiguille (8) de la lancette (2) pour piquer soit mobile au niveau de l'emplacement de piqûre et de mesure et la zone de mesure (7) de l'appareil d'analyse pour mesurer soit mobile au niveau de l'emplacement de piqûre et de mesure.

5. Agencement selon l'une quelconque des revendications 1 ou 2, **caractérisé en ce que** l'appareil d'analyse (1) et la lancette (2) sont maintenus de manière pivotante autour d'un axe de rotation vertical (12) au moyen d'un élément de maintien (9A) dans le dispositif de maintien (3) sous le logement (3B) de sorte qu'une pointe d'aiguille (8) de la lancette (2) et une zone de mesure (7) de l'appareil d'analyse (1) puissent être associés à l'emplacement de piqûre et de mesure défini, un évidement (6A) étant associé en tant qu'emplacement de piqûre et de mesure défini sur le côté inférieur (13) du logement (3A).

6. Agencement selon la revendication 3, **caractérisé en ce que** le logement (3B) est disposé de manière réglable en hauteur sur le dispositif de maintien (3) par des barres de support (15, 16) réglables en longueur de sorte que l'emplacement de piqûre et de mesure soit mobile au niveau de la pointe d'aiguille (8) de la lancette (2) pour piquer et au niveau de la zone de mesure (7) de l'appareil d'analyse (1) pour mesurer.

7. Agencement selon l'une quelconque des revendications précédentes, **caractérisé en ce que** l'axe de rotation (12) de l'élément de maintien (9, 9A) est orienté de telle sorte que l'emplacement de piqûre et de mesure respectivement le logement (6, 6A), la zone de mesure (7) et la pointe d'aiguille (8) soient disposés sur un rayon (R) autour de l'axe de rotation vertical (12).

8. Agencement selon l'une quelconque des revendications précédentes, **caractérisé en ce qu'**un adaptateur d'insertion (10) est associé à l'emplacement de piqûre et de mesure pour le maintien défini de la lancette (2).

9. Agencement selon l'une quelconque des revendications précédentes, **caractérisé en ce qu'**un dispositif de reconnaissance des couleurs et/ou des fluides est associé à l'emplacement de piqûre et de mesure.

10. Agencement selon l'une quelconque des revendications précédentes, **caractérisé en ce qu'**un glucomètre pour mesurer la valeur du glucose est prévu comme appareil d'analyse (1).
